# FASCICULE DE BREVET EUROPEEN

(11) **EP 0 729 749 B1**
(45) Date de publication et mention de la délivrance du brevet: **30.09.1998**
(21) Numéro de dépôt: 96400419.6
(22) Date de dépôt: 28.02.1996
(51) Int. Cl.: A61K 9/48, A61K 31/475

(54) **Compositions pharmaceutiques comprenant des dérivés de dinoréburnaménine**
Pharmazeutische Zusammensetzungen enthaltend Dinoreburnamenine Derivaten
Pharmaceutical compositions containing dinoreburnamenine derivatives

(30) Priorité: 03.03.1995 FR 9502482
(43) Date de publication de la demande: 04.09.1996
(73) Titulaire: HOECHST MARION ROUSSEL, 92800 Puteaux (FR)
(72) Inventeur: Becourt, Philippe, F-91300 Massy (FR); Dubois, Jean-Luc, F-75019 Paris (FR); Metziger, Pierre, F-67450 Lampertheim (FR)
(74) Mandataire: Vieillefosse, Jean-Claude

(56) Documents cités:
- EP-A- 0 317 427
- EP-A- 0 427 998
- FR-A- 2 381 048

## Description

L'invention concerne de nouvelles compositions pharmaceutiques comprenant comme principe actif des dérivés substitués de 20,21-dinoréburnaménine.

Comme principe actif on utilise préférentiellement des produits décrits dans le brevet US 5,093,337.

Ces produits ainsi que leurs sels d'addition présentent d'intéressantes propriétés pharmacologiques, et notamment des propriétés nootropes, anti-dépressives, protectrices neuronales, anti-anoxiques ou anti-ischémiques.

Certains produits présentent une affinité pour les récepteurs alpha 2 adrénergiques. Ils peuvent être utilisés pour le traitement de la maladie d'Alzheimer.

Ces principes actifs sont des composés instables et très sensibles à l'humidité et à l'oxydation. De plus, le principe actif s'est montré incompatible avec la plupart des excipients normalement utilisés dans la formulation des compositions pharmaceutiques.

Il apparaissait donc judicieux de trouver une nouvelle formulation pour ces principes actifs leur assurant une meilleure stabilité.

L'invention concerne notamment une capsule, plus spécifiquement une capsule de gélatine molle constituée d'une enveloppe de capsule et d'un mélange de remplissage de capsule comprenant le principe actif.

Selon l'invention on prépare une association de type suspension ou solution du principe actif avec une ou plusieurs matières du groupe formé par les huiles minérales.

L'enveloppe des capsules est constituée de gélatine, et éventuellement d'autres additifs comme des colorants, un plastifiant ou des sucres. L'enveloppe peut contenir une petite quantité d'eau. On peut utiliser aussi des huiles de silicone et/ou de glycérol dans cette enveloppe.

Le mélange de remplissage de capsule comporte le principe actif, l'huile minérale ainsi que éventuellement, d'autres additifs choisis parmi des substances connues n'altérant pas le principe actif.

L'invention concerne aussi un procédé pour préparer de telles capsules de gélatine molle avec le principe actif.

En principe, la préparation de capsules de gélatine est bien connue. Elle est par exemple décrite en détail dans une publication de Leon Lachmann de 1976 (The Theory and Practice of Industrial Pharmacy (Théorie et pratique de la pharmacie industrielle) Lea et Febiger, Philadelphie, 2e édition, P. 404 - 420, 1976).

Ce document enseigne que la nature des matériaux pour le remplissage de capsules molles est limitée essentiellement à trois catégories, à savoir
a) les supports non miscibles à l'eau,
b) les supports non volatils ou
c) les supports légèrement volatils comme les huiles, les graisses, les huiles éthérées, les hydrocarbures chlorés, les esters, les éthers, les alcools et acides organiques supérieurs.

Le brevet DE-A-33 07 353 décrit un procédé pour préparer des capsules de gélatine molle contenant du polyéthylèneglycol, où le polyéthylèneglycol ne peut être encapsulé de façon fiable que si l'on fixe des paramètres bien définis concernant à la fois l'enveloppe des capsules et les matières du mélange de remplissage des capsules. Les problèmes concernant l'encapsulage peuvent être expliqués par le fait que les charges contenant du polyéthylèneglycol ont des effets prononcés d'échanges avec l'enveloppe des capsules et on ne peut garantir ainsi la stabilité au stockage des capsules.

Le brevet US 2 580 683 (délivré en 1952) décrit des capsules de gélatine qui sont remplies de solutions très concentrées de matières hygroscopiques, comme du sirop de glucose. Le mélange de remplissage des capsules est si concentré en matière hygroscopique que le mélange de remplissage des capsules ne peut plus détruire l'enveloppe des capsules.

La demande EP-A 120 248 décrit des capsules de gélatine molle comportant du polyéthylèneglycol et des procédés pour les préparer. On utilise alors des enveloppes habituelles de capsules. Le mélange de remplissage des capsules est constitué de solutions ou de suspensions de la matière active dans du polyéthylèneglycol.

La présente invention a particulièrement pour objet des capsules molles constituées d'une enveloppe de capsule et d'un mélange de remplissage de capsule, caractérisées en ce que
a) l'enveloppe de capsule contient de la gélatine et éventuellement un plastifiant et d'autres additifs,
b) le mélange de remplissage de capsule contient un composé de formule (I) ou un sel d'addition avec les acides minéraux ou organiques pharmaceutiquement acceptables dudit composé de formule (I) : 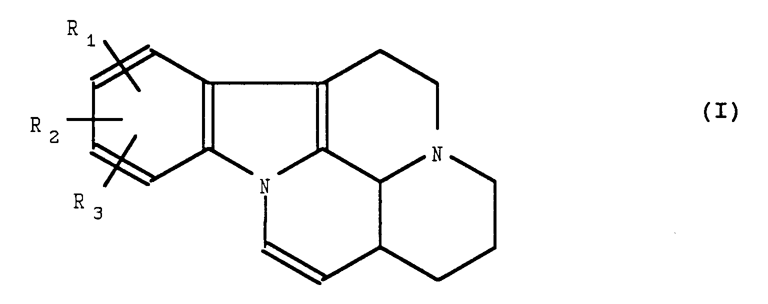 dans laquelle R₁, R₂ et R₃, identiques ou différents, représentent un atome d'hydrogène, un atome d'halogène, un radical alkyle ou alcoxy renfermant de 1 à 5 atomes de carbone, un radical hydroxy, trifluorométhyle, nitro, amino, alkylamino, dialkylamino dans lequel le radical alkyle renferme de 1 à 5 atomes de carbone, un radical acylamino dans lequel le radical acyle représente le reste d'un acide aliphatique renfermant jusqu'à 6 atomes de carbone, et
c) le mélange de remplissage de capsule comporte aussi une ou plusieurs matières du groupe formé par les huiles minérales.

Dans les capsules molles selon la présente invention, l'enveloppe de capsule contient de préférence de la gélatine et un plastifiant choisi parmi les substances connues pour les enveloppes habituelles de capsules.

L'invention a plus particulièrement pour objet les capsules molles telles que définies ci-dessus dans lesquelles le mélange de remplissage de capsule contient un composé de formule (I) ou un sel d'addition pharmaceutiquement acceptable dudit composé de formule (I) dans laquelle R₁ et R₂ représentent des atomes d'hydrogène et R₃ représente un radical alkyle renfermant de 1 à 5 atomes de carbone, et notamment un radical méthyle ou éthyle.

Le radical R₃ se trouve préférentiellement en position 11 du système hétérocyclique.

L'invention a tout particulièrement pour objet une capsule molle, qui contient un composé de formule (II) ou un sel d'addition avec les acides minéraux ou organiques pharmaceutiquement acceptables dudit composé :

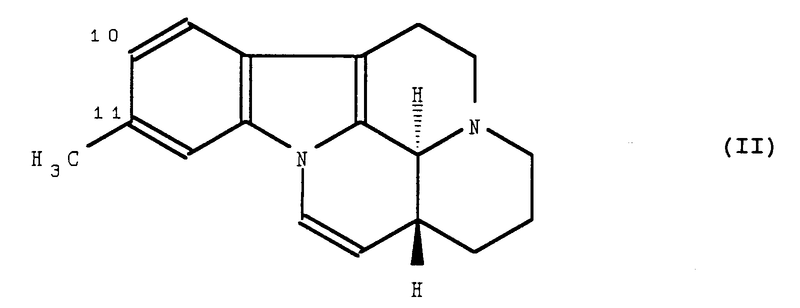

Comme sel d'addition le fumarate ou le maléate du composé (II) ((3-alpha)-11-méthyl-20,21-dinoréburnaménine) est préféré.

Le mélange de remplissage de capsule contient, outre le principe actif de formule (I) ou (II), une ou plusieurs matières du groupe formé par les huiles minérales. Le mélange de remplissage ne contient pas d'eau, ni d'autres solvants qui pourraient altérer le principe actif.

L'invention a notamment pour objet des capsules molles telles que définies ci-dessus dans lesquelles le mélange de remplissage de capsule comprend un composé de formule (I) et une ou plusieurs matières du groupe formé par la vaseline, l'huile de vaseline et l'huile de silicone, de préférence une combinaison de vaseline avec de l'huile de vaseline et tout particulièrement celles dans lesquelles le mélange de remplissage de capsule contient de 1 à 20% en poids de maléate ou de fumarate de (3α)-11-méthyl 20,21-dinoréburnaménine.

L'invention a également pour objet un procédé de préparation d'une capsule molle constituée d'une enveloppe de capsule et d'un mélange de remplissage de capsule, caractérisé en ce que :
a) l'on prépare un mélange pour l'enveloppe de capsule qui contient de la gélatine et éventuellement un plastifiant et d'autres additifs,
b) l'on prépare le mélange de remplissage de capsule qui contient un composé de formule (I) telle que définie ci-dessus ou un sel d'addition avec les acides minéraux ou organiques pharmaceutiquement acceptables dudit composé de formule (I) et une ou plusieurs matières du groupe formé par les huiles minérales, et
c) l'on forme et remplit l'enveloppe avec le mélange de remplissage.

Selon le procédé de l'invention on prépare une solution ou suspension du principe actif avec une ou plusieurs huiles minérales.

Après le remplissage avec la suspension ou solution de principe actif dans l'huile minérale et le séchage habituels des capsules, on obtient des compositions pharmaceutiques qui sont immédiatement utilisables.

Comme composition pharmaceutique selon l'invention on préfère une capsule orale de gélatine molle telle que définie ci-dessus.

Les capsules de gélatine molle ont une importance particulière car elles sont notamment utilisées dans le secteur pharmaceutique pour être avalées sans mâcher. Il est aussi possible de préparer des capsules molles destinées à être mâchées. En outre, il est aussi possible en principe, au moyen des additifs pour les enveloppes de capsules, de préparer des capsules de gélatine dure qui sont stables pendant longtemps au stockage malgré leurs mélanges de remplissage sensibles. Des moyens pour stabiliser l'enveloppe de la capsule molle sont décrits par exemple dans le brevet US 4,804,542.

L'invention concerne aussi la combinaison d'une enveloppe stabilisée par l'ajout d'additifs (comme les amidons) avec un mélange de remplissage qui contient le principe actif et l'huile minérale. On a ainsi constaté que l'effet d'échange entre l'enveloppe de capsule et l'environnement est considérablement limité. Les nouvelles capsules sont considérablement moins exposées à un collage et à un ramolissement, ce qui peut s'expliquer par une plus faible reprise d'humidité en provenance de l'environnement.

Pour mettre en oeuvre le procédé de l'invention de façon à produire des capsules de gélatine avec le produit actif, les additifs sont ajoutés à la décoction de gélatine sous forme d'une suspension ou d'une solution.

La posologie usuelle, variable selon le principe actif utilisé, le sujet traité et l'affection en cause, peut être, par exemple de 0,1 à 200 mg, préférentiellement de 0,5 à 50 mg, par jour par voie orale. Une capsule molle contient par exemple de 0,5 à 50 mg du principe actif.

La préparation du principe actif est décrite en détail dans le brevet US 5,093,337.

Des exemples de préparation de la capsule molle avec le produit de formule (I) sont donnés ci-après.

### Exemple 1

### Préparation d'une capsule molle avec le maléate de ((3-alpha) 11-méthyl-20,21-dinoréburnaménine

A) Le liquide médicamenteux (mélange de remplissage) avec 1 pour-cent de principe actif est réalisé de la façon suivante :
   5,0 g du maléate de ((3-alpha) 11-méthyl-20,21-dinoréburnaménine sont dispersés mécaniquement ou par un autre moyen approprié dans un mélangeur et forment un mélange homogène dans 295 g de vaseline (voir Pharmacopée Française, X, 1990) et 200 g d'huile de vaseline fluide (voir Pharmacopée Européenne, 2nd Ed. et Pharmacopée Française, X, 1990).
B) Parallèlement 263,5 g de gélatine (voir Pharmacopée Européenne, 2nd Ed.) sont intimement mélangés à 102 g de glycérol et 59,5 g d'huile de silicone (par exemple Dimethicones 1000 de Rhône Poulenc Rorer).
C) Les capsules sont réalisées selon le procédé par injection et soudure simultanées. Le mélange de gélatine maintenu à une température de 60° à 70°C est déposé sur des tambours rotatifs refroidis intérieurement en un film de gélatine qui se solidifie.
   Les deux films formés symétriquement passent entre deux matrices alvéolées en demi capsule où le liquide médicamenteux (constituant le mélange de remplissage) est injecté à l'aide d'une pompe volumétrique ajustée précisément à 117 microlitres (correspondant à 100 mg) pour des capsules ovales. Le liquide repousse le film de gélatine dans les alvéoles. La pression exercée par les deux cylindres combinée à une température de chauffage adéquate est telle que la gélatine est soudée et découpée autour de chaque alvéole. Les capsules sont séchées en étuve ventilée ou tout autre moyen approprié.

### Exemple 2

### Préparation d'une capsule molle avec le maléate de ((3-alpha) 11-méthyl-20,21-dinoréburnaménine

A) Le liquide médicamenteux (constituant le mélange de remplissage) avec 20 pour-cent de principe actif est réalisé de la façon suivante :
   100,0 g du maléate de ((3-alpha) 11-méthyl-20,21-dinoréburnaménine sont dispersés mécaniquement ou par un autre moyen approprié dans un mélangeur et forment un mélange homogène avec 200 g de vaseline (voir Pharmacopée Française, X, 1990) et de 200 g d'huile de vaseline fluide (voir Pharmacopée Européenne, 2nd Ed. et Pharmacopée Française, X, 1990).
B) Parallèlement 263,5 g de gélatine (voir Pharmacopée Européenne, 2nd Ed.) sont intimement mélangés à 102 g de glycérol et 59,5 g d'huile de silicone (par exemple Dimethicones 1000 de Rhône Poulenc Rorer).
C) Les capsules sont réalisées selon le procédé par injection et soudure simultanées.

Le mélange de gélatine maintenu à une température de 60°C est déposé sur des tambours rotatifs refroidis intérieurement en un film de gélatine qui se solidifie.

Les deux films formés symétriquement passent entre deux matrices alvéolées en demi capsule où le liquide médicamenteux (le mélange de remplissage) est injecté à l'aide d'une pompe volumétrique ajustée précisément à 117 microlitres (correspondant à 100 mg) pour des capsules ovales. Le liquide repousse le film de gélatine dans les alvéoles. La pression exercée par les deux cylindres combinée à une température de chauffage adéquate est telle que la gélatine est soudée et découpée autour de chaque alvéole. Les capsules sont séchées en étuve.

La stabilité des capsules molles de tailles et formes différentes selon les exemples 1 et 2 a été testée à des températures différentes.
Après 1 an à température de 25°C aucune altération significative des capsules molles ni du principe actif (au seuil de détection 0,2 %) n'a été mise en évidence.

## Revendications

1. Capsules molles constituées d'une enveloppe de capsule et d'un mélange de remplissage de capsule, caractérisées en ce que
a) l'enveloppe de capsule contient de la gélatine et éventuellement un plastifiant et d'autres additifs,
b) le mélange de remplissage de capsule contient un composé de formule (I) ou un sel d'addition avec les acides minéraux ou organiques pharmaceutiquement acceptables dudit composé de formule (I) : 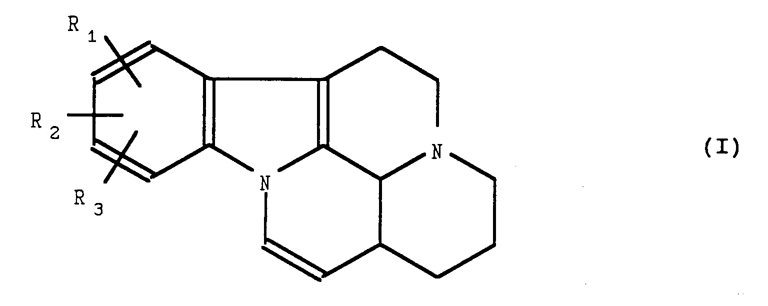 dans laquelle R₁, R₂ et R₃, identiques ou différents, représentent un atome d'hydrogène, un atome d'halogène, un radical alkyle ou alcoxy renfermant de 1 à 5 atomes de carbone, un radical hydroxy, trifluorométhyle, nitro, amino, alkylamino, dialkylamino dans lequel le radical alkyle renferme de 1 à 5 atomes de carbone, un radical acylamino dans lequel le radical acyle représente le reste d'un acide aliphatique renfermant jusqu'à 6 atomes de carbone, et
c) le mélange de remplissage de capsule comporte aussi une ou plusieurs matières du groupe formé par les huiles minérales.

2. Capsules molles selon la revendication 1, caractérisées en ce que le mélange de remplissage de capsule contient un composé de formule (I) ou un sel d'addition pharmaceutiquement acceptable dudit composé de formule (I) dans laquelle R₁ et R₂ représentent des atomes d'hydrogène et R₃ représente un radical alkyle renfermant de 1 à 5 atomes de carbone.

3. Capsules molles selon la revendication 1 ou 2, caractérisées en ce que le mélange de remplissage de capsule contient un composé de formule (I) ou un sel d'addition pharmaceutiquement acceptable dudit composé de formule (I) dans laquelle R₁ et R₂ représentent des atomes d'hydrogène et R₃ représente un radical méthyle en position 11.

4. Capsules molles selon l'une des revendications 1 à 3, caractérisées en ce que le mélange de remplissage de capsule comprend un composé de formule (I) et une ou plusieurs matières du groupe formé par la vaseline, l'huile de vaseline et l'huile de silicone.

5. Capsules molles selon l'une des revendications 1 à 4, caractérisées en ce que le mélange de remplissage de capsule comprend un composé de formule (I) et une combinaison de vaseline avec de l'huile de vaseline.

6. Capsules molles selon la revendication 5,caractérisées en ce que le mélange de remplissage contient de 1 à 20% en poids de maléate ou de fumarate de (3α)11-méthyl 20,21-dinoréburnaménine.

7. Procédé de préparation d'une capsule molle selon la revendication 1 constituée d'une enveloppe de capsule et d'un mélange de remplissage de capsule, caractérisé en ce que
a) l'on prépare un mélange pour l'enveloppe de capsule qui contient de la gélatine et éventuellement un plastifiant et d'autres additifs,
b) l'on prépare le mélange de remplissage de capsule qui contient un composé de la formule (I) telle que définie à la revendication 1, 2 ou 3 ou un sel d'addition avec les acides minéraux ou organiques pharmaceutiquement acceptables dudit composé de formule (I) et une ou plusieurs matières du groupe formé par les huiles minérales, et
c) l'on forme et remplit l'enveloppe avec le mélange de remplissage.

## Claims

1. Soft capsules constituted by a capsule casing and a mixture for filling the capsule, characterized in that
a) the capsule casing contains gelatin and optionally a plasticizer and other additives,
b) the mixture for filling the capsule contains a compound of formula (I) or an addition salt with pharmaceutically acceptable mineral or organic acids of said compound of formula (I): 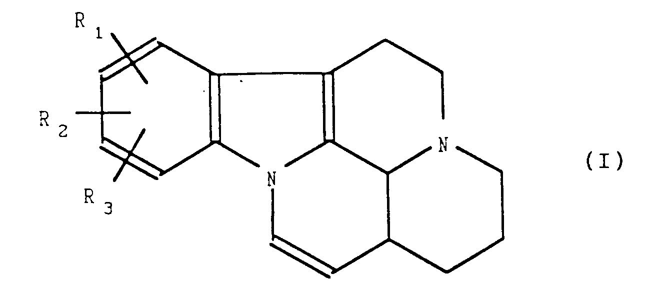 in which R₁, R₂ and R₃, identical or different, represent a hydrogen atom, a halogen atom, an alkyl or alkoxy radical containing 1 to 5 carbon atoms, one of the following radicals: hydroxy, trifluoromethyl, nitro, amino, alkylamino, dialkylamino in which the alkyl radical contains 1 to 5 carbon atoms, an acylamino radical in which the acyl radical represents the remainder of an aliphatic acid containing up to 6 carbon atoms, and
c) the mixture for filling the capsule also contains one or more substances from the group formed by mineral oils.

2. Soft capsules according to claim 1, characterized in that the mixture for filling the capsule contains a compound of formula (I) or a pharmaceutically acceptable addition salt of said compound of formula (I) in which R₁ and R₂ represent hydrogen atoms and R₃ represents an alkyl radical containing 1 to 5 carbon atoms.

3. Soft capsules according to claim 1 or 2, characterized in that the mixture for filling the capsule contains a compound of formula (I) or a pharmaceutically acceptable addition salt of said compound of formula (I) in which R₁ and R₂ represent hydrogen atoms and R₃ represents a methyl radical in position 11.

4. Soft capsules according to one of claims 1 to 3, characterized in that the mixture for filling the capsule contains a compound of formula (I) and one or more substances from the group formed by vaseline, vaseline oil and silione fluid.

5. Soft capsules according to one of claims 1 to 4, characterized in that the mixture for filling the capsule contains a compound of formula (I) and a combination of vaseline with vaseline oil.

6. Soft capsules according to claim 5, characterized in that the filling mixture contains 1 to 20% by weight of (3α)11-methyl 20,21-dinoreburnamenine maleate or fumarate.

7. Preparation process for a soft capsule according to claim 1 constituted by a capsule casing and a mixture for filling the capsule, characterized in that
a) a mixture for the capsule casing is prepared which contains gelatin and optionally a plasticizer and other additives,
b) the mixture for filling the capsule is prepared which contains a compound of formula (I) as defined in claim 1, 2 or 3 or an addition salt with pharmaceutically acceptable mineral or organic acids of said compound of formula (I) and one or more substances from the group formed by mineral oils, and
c) the casing is formed and filled with the filling mixture.

## Patentansprüche

1. Weiche Kapseln, bestehend aus einer Kapselumhüllung und einer Mischung zur Kapselfüllung, dadurch gekennzeichnet, daß
a) die Kapselumhüllung Gelatine und gegebenenfalls einen Weichmacher sowie andere Zusatzstoffe enthält,
b) die Mischung zur Kapselfüllung eine Verbindung der Formel (I) oder ein Additionssalz mit pharmazeutisch akzeptablen Mineralsäuren oder organischen Säuren der genannten Verbindung der Formel (I) 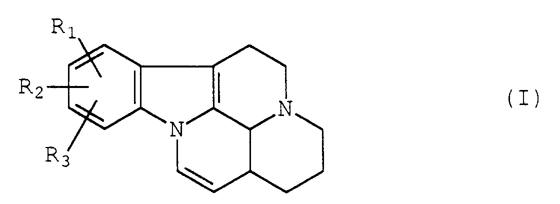 enthält, in der R₁, R₂ und R₃, gleich oder verschieden, ein Wasserstoffatom, ein Halogenatom, einen Rest Alkyl oder Alkoxy mit 1 bis 5 Kohlenstoffatomen, einen Rest Hydroxy, Trifluormethyl, Nitro, Amino, Alkylamino, Dialkylamino, bei dem der Rest Alkyl 1 bis 5 Kohlenstoffatome umfaßt, einen Rest Acylamino, bei dem der Rest Acyl den Rest einer aliphatischen Säure mit bis zu 6 Kohlenstoffatomen bedeutet, darstellen, und
c) die Mischung zur Kapselfüllung außerdem einen oder mehrere Stoffe aus der durch die Mineralöle gebildeten Gruppe umfaßt.

2. Weiche Kapseln nach Anspruch 1, dadurch gekennzeichnet, daß die Mischung zur Kapselfüllung eine Verbindung der Formel (I) oder ein pharmazeutisch akzeptables Additionssalz der genannten Verbindung der Formel (I) enthält, worin R₁ und R₂ Wasserstoffatome sind und R₃ einen Rest Alkyl mit 1 bis 5 Kohlenstoffatomen darstellt.

3. Weiche Kapseln nach Anspruch 1 oder 2, dadurch gekennzeichnet, daß die Mischung zur Kapselfüllung eine Verbindung der Formel (I) oder ein pharmazeutisch akzeptables Additionssalz der genannten Verbindung der Formel (I) enthält, worin R₁ und R₂ Wasserstoffatome sind und R₃ einen Rest Methyl in Position 11 darstellt.

4. Weiche Kapseln nach einem der Ansprüche 1 bis 3, dadurch gekennzeichnet, daß die Mischung zur Kapselfüllung eine Verbindung der Formel (I) und einen oder mehrere Stoffe aus der durch Vaseline, Vaselineöl und Siliconöl gebildeten Gruppe umfaßt.

5. Weiche Kapseln nach einem der Ansprüche 1 bis 4, dadurch gekennzeichnet, daß die Mischung zur Kapselfüllung eine Verbindung der Formel (I) und eine Kombination von Vaseline mit Vaselineöl umfaßt.

6. Weiche Kapseln nach Anspruch 5, dadurch gekennzeichnet, daß die Mischung zur Kapselfüllung 1 Gew.-% bis 20 Gew.-% Maleat oder Fumarat von (3α)11-Methyl-20,21-dinoreburnamenin umfaßt.

7. Verfahren zur Herstellung einer weichen Kapsel nach Anspruch 1, bestehend aus einer Kapselumhüllung und einer Mischung zur Kapselfüllung, dadurch gekennzeichnet, daß man
a) eine Mischung für die Kapselumhüllung herstellt, die Gelatine und gegebenenfalls einen Weichmacher sowie andere Zusatzstoffe enthält,
b) die Mischung zur Kapselfüllung herstellt, die eine wie in Anspruch 1, 2 oder 3 definierte Verbindung der Formel (I) oder ein Additionssalz mit pharmazeutisch akzeptablen Mineralsäuren oder organischen Säuren der genannten Verbindung der Formel (I) und einen oder mehrere Stoffe aus der durch die Mineralöle gebildeten Gruppe umfaßt, und
c) die Umhüllung formt und mit der Mischung zur Kapselfüllung füllt.
